(19) Europäisches Patentamt
European Patent Office
Office européen des brevets

(11) Publication number: 0 116 205
A1

(12) # EUROPEAN PATENT APPLICATION

(21) Application number: 83307172.3

(51) Int. Cl.³: C 07 D 233/60

(22) Date of filing: 24.11.83

(30) Priority: 01.12.82 US 445787

(43) Date of publication of application: 22.08.84 Bulletin 84/34

(84) Designated Contracting States: AT BE CH DE FR GB IT LI LU NL SE

(71) Applicant: PFIZER INC.
235 East 42nd Street
New York, N.Y. 10017(US)

(72) Inventor: Cue, Berkeley W.
266 Stoddards Wharf Road
Gales Ferry Connecticut 06335(US)

(74) Representative: Graham, Philip Colin Christison et al,
Pfizer Limited Ramsgate Road
Sandwich, Kent CT13 9NJ(GB)

(54) 4(5)-Acetyl-2-methylimidazole process.

(57) Process for making 4(5)-acetyl-2-methylimidazole, a valuable intermediate, from 2-methylimidazole-4(5)-carboxaldehyde, the 1-position of which is protected by a benzyl group which may be unsubstituted or substituted. The process comprises a Grignard reaction followed by deblocking and oxidation, or by oxidation then deblocking. The oxidation is effected in a reaction-inert solvent with manganese dioxide.

EP 0 116 205 A1

# 4(5)-ACETYL-2-METHYLIMIDAZOLE PROCESS

This invention relates to an improved process for making 4(5)-acetyl-2-methylimidazole, a valuable intermediate. More particularly, it relates to preparation of said intermediate by Grignard reaction of methyl magnesium chloride on 2-methylimidazole-4(5)-carboxaldehyde, the 1-position of which is suitably protected, followed by removal of the protecting group and oxidation, or by oxidation followed by removal of the protecting group.

Known methods for making 4(5)-acetyl-2-methylimidazole are described in EP Publication No. 50,458A, published April 28, 1982. They comprise irradiation of 1-acetyl-2-methylimidazole with ultraviolet light, and reaction of a 3-halo-4-n-alkoxy-3-buten-2-one with acetamidine, or a salt thereof, in the presence of a base such as triethylamine in a reaction-inert solvent. The yields afforded by these two processes are relatively low, on the order of from 40-50% to 20-30%, respectively.

The preparation of secondary alcohols from aldehyde via the Grignard reaction is well known in organic chemistry (Fieser and Fieser, "Organic Chemistry", D. C. Heath and Company, Boston, Mass., 1944, pages 118-119). Also well known is the oxidation of secondary alcohols to ketones and the use of manganese dioxide as an oxidizing agent in general and for conversion of alcohols to ketones (Fieser and Fieser, "Reagents for Organic Synthesis", John Wiley & Sons, Inc., N.Y., 1967, pp. 636-643).

It has now been found that 4(5)-acetyl-2-methylimidazole can be prepared by the reactions outlined below which are not only convenient and readily adaptable to large scale operation but are also productive of high quality product in yields much higher than those of the prior art processes.

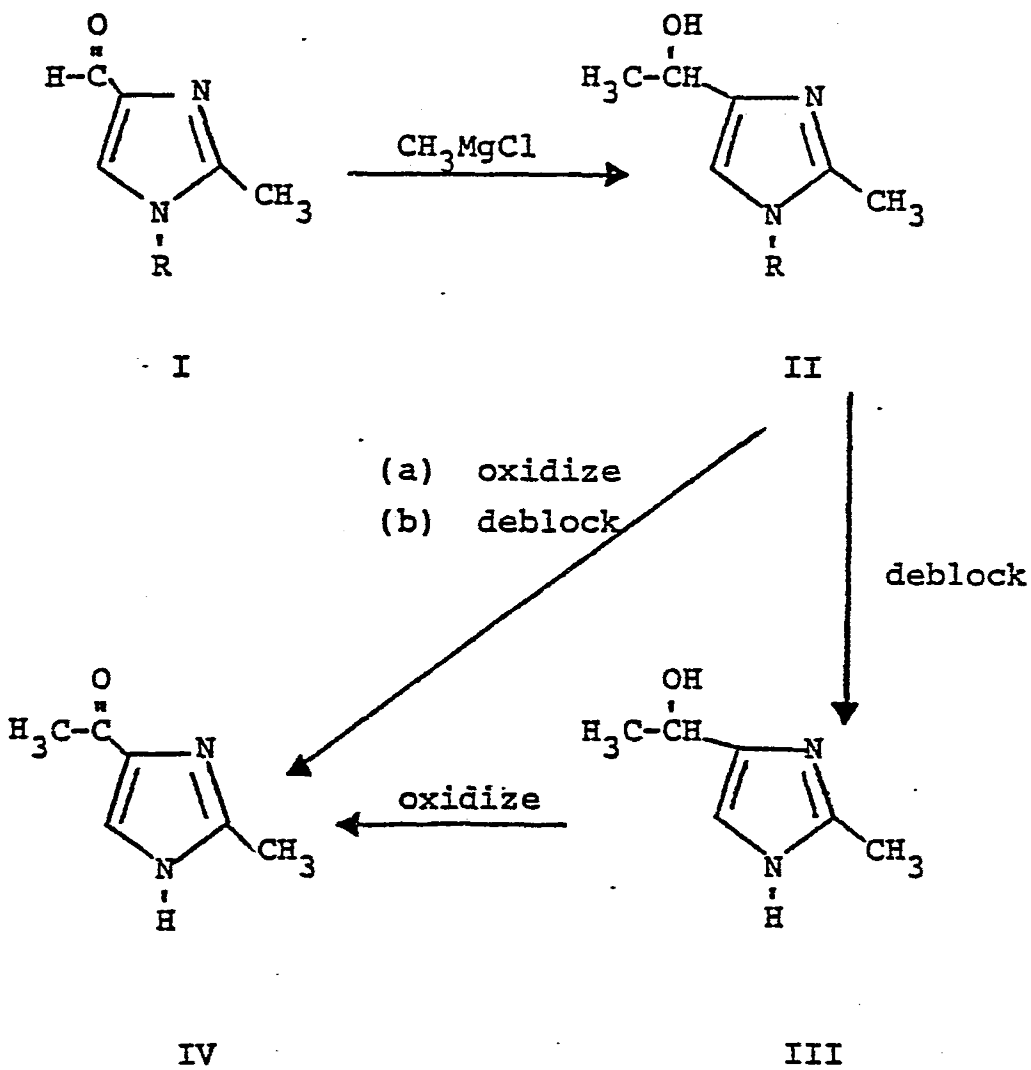


I II

IV III

In the above formulae, R represents a protecting group and advantageously a benzyl or mono-substituted benyl group having formula (a):

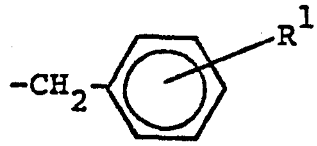

(a)

wherein $R^1$ is hydrogen, chloro, bromo, fluoro, $(C_{1-4})$-alkyl, $(C_{1-4})$alkoxy or phenyl.

4(5)-Acetyl-2-methylimidazole is an intermediate for the preparation of 2-guanidino-4-(2-methyl-4(5)-imidazolyl)thiazole, a histamine $H_2$ antagonist of value in the treatment of gastric hyperacidity and peptic ulcers as is described in EP Publication No. 50,458A, published April 28, 1982.

The improved process of this invention begins with 2-methylimidazole-4(5)-carboxaldehyde, the 1-position of which is protected by a benzyl group, as such group is defined under formula (a). The preferred protecting group is benzyl because of its relative ease of removal under relatively mild conditions.

The formula I compound wherein R is benzyl is a known compound. The remaining compounds of formula I, those wherein R is substituted benzyl are new compounds. Said new compounds are prepared from 2-methylimidazole in the same manner as is 1-benzyl-2-methylimidazole-4-carboxaldehyde. The procedure comprises aralkylating the sodium salt of 2-methylimidazole with the appropriate benzyl chloride (or bromide) in dimethylformamide according to the procedure described by Godefroi, J. Org. Chem. 33, 860-861 (1968). The thus-produced 2-methylimidazole, the 1-position of which is protected, is then hydroxymethylated in an acetic acid-sodium acetate buffered medium according to the procedure of Godefroi *et al*., Rec. trav. chim. Pays Bas 91, 1383-1392 (1972).

The 1-(R-substituted)-4-hydroxymethyl-2-methylimidazole thus produced is then oxidized by means of lead tetra-acetate in pyridine to the corresponding 1-(R-substituted)-2-methylimidazole-4-carboxaldehyde (I).

The nature of the protecting group R is not critical to this invention since its importance resides in its ability to protect the imidazole N-H bond from undesired reactions coupled with its subsequent ease of removal to regenerate said N-H bond under conditions which do not cause reactions at other sites of the protected compound or of the deblocked compound produced therefrom. The selection and

identification of appropriate protecting groups can easily and readily be made by one skilled in the art. The suitability and effectiveness of a group as a N-H protecting group are determined by employing said group in the above reaction sequence.

In addition to benzyl and substituted derivatives thereof as protecting groups, benzhydryl, benzyloxymethyl, 1- and 2-naphthylmethyl and 9-fluorenyl which are readily removed by catalytic hydrogenolysis over Pd/C; and trityl, substituted trityl and trialkylsilyl groups such as trimethylsilyl which are easily removed by mild acid hydrolysis can be used as protecting groups.

The protected 2-methylimidazole-4-carboxaldehyde (I) is then reacted with methyl magnesium chloride (Grignard reaction) in a reaction-inert solvent such as tetrahydrofuran, dioxane, diethyl ether to produce an alcohol of formula II.

The formula II compound is then deblocked to provide the formula III compound. Deblocking is accomplished by catalytic hydrogenolysis using palladium, and especially palladium-on-carbon, as catalyst. The reaction is conducted in a reaction-inert solvent such as methanol or ethanol over Pd/C as catalyst. The presence of acetic acid is generally advantageous in the deblocking step when the formula II compound is oxidized to the acetyl derivative prior to deblocking.

The hydrogen pressure used is not critical but can vary over a wide range such as from subatmospheric to superatmospheric pressures, e.g. from 0.5 to 2000 atmospheres. However, from a practical standpoint pressures of from about 1 to about 10 atmospheres (1.03 to 10.3 kg/sq. cm) and preferably from about 1 to about 3 atmospheres (1.03 to 3.10 kg/sq. cm) are used. The temperature of hydrogenolysis is not critical but can vary from about 20°C to about 150°C. Temperatures of from 20°C to 100°C and preferably from 20°C to 50°C are used.

The deblocked alcohol compound of formula III is converted to the acetyl compound by oxidation in a reaction-inert solvent using as oxidizing agent manganese dioxide. The oxidation is carried out at temperatures ranging from ambient temperature to 100°C and desirably at from 25° to 50°C since this range represents a balance between reaction times, yields and energy consumption. Representative reaction-inert solvents for this oxidiation are dioxane, tetrahydrofuran, benzene, toluene, chlorinated hydrocarbons, such as chloroform and methylene chloride.

The manganese dioxide and formula III compound are generally reacted in molar ratios of from about 2:1 to about 4:1. Higher ratios can be used but offer no advantage and are undesirable from an economic viewpoint.

Alternatively, the blocked compound (formula II) is oxidized according to the above-described procedures; and the 1-benzyl-2-acetyl-(4)5-methylimidazole compound, then deblocked according to the procedures described above.

## EXAMPLE I

### I-Benzyl-2-Methylimidazole

To a slurry of 2.4 g (0.1 mole) of sodium hydride in 50 ml of dimethylformamide under a nitrogen atmosphere was added, with stirring, 8.2 g (0.1 mole) of 2-methylimidazole. A slow exothermic reaction occurred, the temperature reaching 43°C. When the exotherm subsided, the reaction was warmed on a steam bath to 70°-75°C for a half-hour and then at 95°C for 15 minutes to complete the reaction as evidenced by cessation of gas evolution. It was then cooled to 68°C and 12.7 g (0.1 mole) of benzyl chloride added dropwise. An exothermic reaction occurred, the temperature reaching 95°C. After stirring for a half-hour following completion of addition, the reaction was poured into 600 ml of water and the product extracted with ethyl acetate (2 x 200 ml). The combined extracts were washed successively with water (1 x 400 ml), saturated aqueous sodium chloride solution (1 x 100 ml), then with 6N HCl (1 x 50 ml). The HCl wash was extracted with ether (1 x 25 ml) and then made basic by addition of sodium hydroxide. The yellow oil which separated was extracted into ether, the extract dried ($MgSO_4$) and evaporated under reduced pressure to give a pale yellow oil. Yield, 11.5 g (60.5%). NMR indicates the compound was obtained as the mono hydrate. It was used as is in the hydroxymethylation reaction.

Repetition of this procedure but using the appropriate substituted benzyl halide (chloride or bromide) in place of benzyl chloride affords the following compounds:

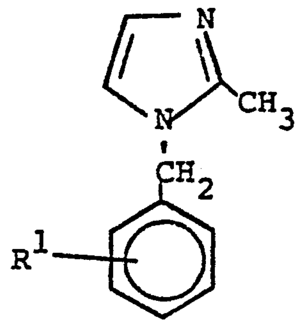

| $\underline{R^1}$ | $\underline{R^1}$ | $\underline{R^1}$ |
|---|---|---|
| 2-Cl | 2-$OCH_3$ | 2-$CH_3$ |
| 3-Cl | 4-O-n-$C_4H_9$ | 3-$CH_3$ |
| 4-Cl | 2-$OC_2H_5$ | 4-$CH_3$ |
| 4-Br | 2-F | 4-n-$C_4H_9$ |
| 4-$OCH_3$ | 4-F | 3-n-$C_3H_7$ |
| | | 4-$C_6H_5$ |

## EXAMPLE 2

### 1-Benzyl-4-Hydroxymethyl2-Methylimidazole

A mixture of 8.5 g (0.05 mole) of 1-benzyl-2-methylimidazole monohydrate, 50 ml of 36% formaldehyde, 6 ml of acetic acid and 8.0 g (0.098 mole) of sodium acetate is stirred and heated at reflux for 26 hours. It was then stirred over a week end (about 65 hours) at room temperature and neutralized with solid sodium carbonate. The neutral solution was extracted with ethyl acetate, the extract dried ($MgSO_4$) and evaporated under reduced pressure to an oil. Water (10 ml) and isopropanol (50 ml) were added to the oil, the solution stirred overnight then evaporated under reduced pressure. The oily residue obtained was taken up in water and the solution made strongly basic by addition of solid sodium hydroxide. It was chilled, layered with diethyl ether, and the white solid which formed removed by filtration and air dried. Yield = 1.8 g (18%); M.P. 140-146°C.

It was purified by dissolution in 30 ml of hot (50°C) ethyl acetate and filtration. Concentration of the filtrate to about two-thirds volume, and chilling, afforded 1.3 g of white solid; M.P. 147-151°C. Thin layer chromatography in the system ethyl acetate: methanol:diethylamine (80:10:10) gave a single spot.

EXAMPLE 3

1-Benzyl-2-Methylimidazole-4-Carboxaldehyde

A slurry of 9.0 g (0.446 mole) of 1-benzyl-4-hydroxymethyl-2-methylimidazole (product of Example 2), 750 ml of methylene chloride and 50.0 g (0.575 mole) of manganese dioxide was stirred at room temperature for two hours. It was then filtered, the filter cake washed with methylene chloride and the combined filtrate and wash solutions evaporated under reduced pressure to give an oil. The oil was taken up in 100 ml of diethyl ether, 100 ml of hexane added and the solution seeded with a few crystals of the title compound. Concentration of the solution under a nitrogen sweep with periodic replacement of hexane afforded a crystalline product which was isolated by filtration: 7.2 g, 81% yield. M.P. 57-60°C.

A second crop (0.75 g) was obtained by concentration of the filtrate. M.P. 57-59.5°C. Total yield = 89.4%.

Similarly, the remaining compounds of Example 1 are converted to the respective hydroxymethyl derivatives and then to the respective carboxaldehyde derivatives.

## EXAMPLE 4

### 1-Benzyl-4-(1-Hydroxyethyl)-2-Methylimidazole

To a solution of 7.2 g (0.306 mole) of 1-benzyl-2-methylimidazol-4-carboxaldehyde (title product of Example 3) in 100 ml of tetrahydrofuran was added 15 ml of 2.9M methyl magnesium chloride (0.044 mole) in tetrahydrofuran. A white precipitate formed immediately. The mixture was stirred at room temperature for 30 minutes and then heated with 50 ml of 25% aqueous ammonium chloride solution. The precipitate was filtered off, washed with tetrahydrofuran and air dried. The combined filtrate and wash solutions were dried ($Na_2SO_4$) and concentrated *in vacuo* to a solid residue. The residue was dissolved in 300 ml of boiling ethyl acetate, dried ($Na_2SO_4$) and concentrated to half volume under reduced pressure. The solid which separated upon cooling was filtered off and air dried. Total yield = 7.1 g (90%). M.P. 162.5-167.5°C.

The remaining compounds of Example 3 are converted to the corresponding 1-protected-4-(1-hydroxyethyl)-2-methylimidazoles in like manner.

EXAMPLE 5

4(5)-(1-Hydroxyethyl)-2-Methylimidazole

A Parr shaker was charged with 10.0 g (46.23 mmol) of 1-benzyl-4-(1-hydroxyethyl)-2-methylimidazole (product of Example 4), 60 ml of methanol and 2.0 g of 5% palladium-on-carbon (50% water). Hydrogen gas was introduced to 30 psi (2.04 atmospheres), the mixture heated to 50°C and shaken for 16 hours. It was cooled to 30°C, filtered through diatomaceous earth and the filter cake washed with 10 ml of methanol. Evaporation of the combined filtrate and wash under reduced pressure gave 6.44 g (97% yield) of the title product as an oil.

The product can be crystallized by adding enough tetrahydrofuran to dissolve the oil and stirring the solution at ambient temperature for two hours. The white crystalline solid was collected by filtration and air dried. M.P. 107-111°C.

The 1-(substituted benzyl) derivatives of Example 4 are deblocked in like manner.

## EXAMPLE 6

### 4(5)-Acetyl-2-Methylimidazole

To a refluxing mixture of 1240 g (9.989 moles) of 4(5)-(1-hydroxyethyl)-2-methylimidazole (title product of Example 5) in 10 liters of tetrahydrofuran was added 2200 g (25.293 moles) of manganese dioxide over a period of ten minutes. The mixture was refluxed overnight (18 hours), then filtered hot through diatomaceous earth. The filter cake was washed with 4 liters of tetrahydrofuran.

The combined filtrates and washings from two such reactions were stirred and concentrated at atmospheric pressure to about 6 liter volume at which point the mixture became solid. Ethyl acetate (2 liters) was added, the mixture heated to form a solution and to permit further removal of tetrahydrofuran. When the mixture became solid, an additional 2 liters of ethyl acetate was added and the heating repeated. When the mixture became solid, heating and stirring were discontinued and the mixture cooled overnight. Ethyl acetate (3.8 liters) was added and the solid mass broken up with the aid of a spatula. When it became stirrable, the slurry was heated at 50°C for 3 hours, then cooled at 5°C for one hour and filtered with suction. The yellow filter cake was washed with 1.5 liters of ethyl acetate at 5°C then air dried. Yield = 1887 g (76.08%). M.P. 128-130°C.

EXAMPLE 7

4-Acetyl-1-Benzyl-2-Methylimidazole

A slurry of 6.0 g (0.0278 mole) of 1-benzyl-4(5)-(1-hydroxyethyl)-2-methylimidazole (product of Example 4), 125 ml of tetrahydrofuran and 35.0 g (0.403 mole) of manganese dioxide was stirred at room temperature for a half-hour and then at reflux for one hour. It was then filtered, the filter cake washed with tetrahydrofuran, and the combined filtrate and wash solutions concentrated to an oil. The oil was overlayed with hexane and the mixture stirred overnight. The white solid which formed was filtered off, washed with hexane and air dried. Yield = 4.5 g (75.5%). M.P. = 43-47.5°C.

A second crop (300 mg) was recovered from the mother liquor.

The remaining compounds of Example 4 are oxidized in like manner to produce the corresponding 4-acetyl-1-protected-2-methylimidazoles.

EXAMPLE 8

4(5)-Acetyl-2-methylimidazole

A suspension of 33.9 g (0.156 mole) of 1-benzyl-4-(1-hydroxyethyl)-2-methylimidazole in 1330 ml of tetrahydrofuran was heated to reflux until complete solution occurred. It was then cooled to 50°C and 156.5 g (1.80 moles) of manganese dioxide added. The mixture was refluxed for two hours, cooled to 55°C then filtered and the filtrate concentrated under reduced pressure to an oil. Methanol (500 ml) was added to the oil and the solution concentrated under reduced pressure. This step was repeated once more and the resulting yellow oil dissolved in 417.5 ml of methanol.

The methanol solution was placed in a Parr bottle, 41.7 ml of acetic acid and 33.4 g of palladium-on-charcoal (50% water) added. The bottle was purged with nitrogen and then with hydrogen. Hydrogen at 25 psi (1.70 atmospheres) was introduced and the bottle shaken overnight at room temperature. The bottle was repressurized with hydrogen at 50 psi (3.40 atmospheres) and shaken for an additional five hours. The contents of the bottle were filtered off and the filter cake washed with methanol. The combined filtrate and wash solutions were concentrated *in vacuo* to an oil. Water (35 ml) was added to the oil followed by 95.9 g of potassium carbonate. When foaming had subsided, 200 ml of tetrahydrofuran was added and the mixture stirred for 30 minutes.

The solids were filtered off and washed with tetrahydrofuran. The filtrate plus wash solution separated into two layers. The tetrahydrofuran layer was separated, dried ($K_2CO_3$) and concentrated in vacuo. The solid residue was dissolved in refluxing ethyl acetate, (150 ml), the solution concentrated to half volume then stirred at room temperature over a week end. It was cooled to 5°C, the solid filtered off, washed with cold ethyl acetate and dried in vacuo at 40°C. Yield = 12 g (61.9%). M.P. 127-129°C.

A further 1.8 g of solid was recovered from the mother liquor by concentration thereof. Total yield = 71.2%.

## EXAMPLE 9

### 1-Benzyl-4-(1-Hydroxyethyl)-2-Methylimidazole

A slurry of 15 g (0.744 mole) of 1-benzyl-4-hydroxymethyl-2-methylimidazole in 300 ml of tetrahydrofuran was slowly warmed to 50°-55°C to form a solution. Manganese dioxide (30.1 g) was added in four equal portions at fifteen minute intervals. One hour after completion of addition, the reaction mixture was filtered and the filter cake washed with two 100 ml volumes of tetrahydrofuran. The combined filtrate and wash solutions were dried ($MgSO_4$). To the dried solution under an atmosphere of nitrogen was added 35 ml of 2.9M methylmagnesium chloride in tetrahydrofuran. One-half hour later a second 35 ml portion of methylmagnesium chloride was added, followed a half-hour later by a 17 ml portion. The reaction mixture was stirred for 15 minutes and then treated with 125 ml of 25% aqueous ammonium chloride solution. The white solid was removed by filtration and washed with saturated aqueous ammonium chloride solution. The filtrate and wash solutions were combined, dried ($Na_2SO_4$) and evaporated in vacuo. The residue was taken up in boiling ethyl acetate, decolorized with activated charcoal, and concentrated to a volume of about 150 ml. It was then allowed to cool to room temperature and the title product, which precipitated, filtered off, washed with ethyl acetate and air dried. Yield = 13.9 g (86.3%). M.P. 162°-165°C.

EXAMPLE 10

4(5)-Bromoacetyl-2-methylimidazole Hydrobromide

2.40 g (19.3 mmol) of 4(5)-acetyl-2-methylimidazole was dissolved in 30 ml of 48% hydrogen bromide. To the stirred solution at 25° was added over a 5 minute period 3.36 g (21 mmol) of bromine dissolved in 5 ml 48% hydrogen bromide. The reaction was heated to 70° for 2.5 hours and then concentrated *in vacuo* to a dark oil. A mixture of isopropyl alcohol/ether was added and trituration of the oil gave a solid. This was collected by filtration and washed with ether to give 2.8 g (51%) of the title product, M.P. 181° (dec.); nmr (DMSO-$d_6$) (delta): 8.71 (s, 1H); 4.77 (s, 2H); 2.63 (s, 3H).

EXAMPLE 11

2-Guanidino-4-(2-methyl-4(5)-imidazolyl)thiazole Hydrobromide

2.8 g (9.86 mmol) of 4(5)-bromo-acetyl-2-methylimidazole hydrobromide was dissolved in 10 ml water. Saturated sodium bicarbonate solution was added to pH 10 and the resultant solid was collected by filtration and washed with 15 ml water. The dried free base was heated at reflux in 50 ml acetone. To the refluxing clear acetone solution was added 1.2 g (9.86 mmol) of amidinothiourea. Solution occurred immediately and within a minute a solid began to form. After one hour reflux the slurry was cooled and the solid was collected by filtration and was washed with acetone followed by ether to give 2.37 g (79%) of the title compound, M.P. 158° (dec.); nmr ($DMSO-d_6$) (delta): 7.71 (s shoulder on broad s, 1H); 7.56 (broad s, 4H); 4.32 (s, 1H); 2.51 (s, 3H).

CLAIMS

1. A process for making a compound of the formula

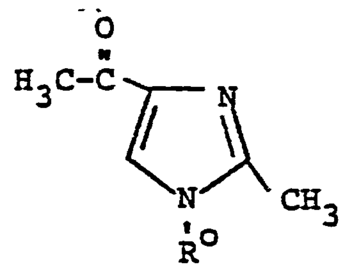

wherein $R^o$ is hydrogen, benzyl or mono-substituted benzyl, wherein the substituent is chloro, bromo, fluoro, $(C_{1-4})$alkyl, $(C_{1-4})$alkoxy or phenyl,

which comprises oxidizing a compound of the formula

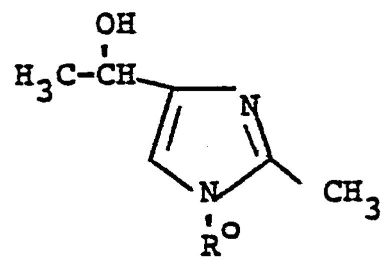

wherein $R^o$ is as defined above in a reaction-inert solvent with manganese dioxide.

2. The process of claim 1 wherein $R^o$ is hydrogen.

3. The process of claim 1 wherein $R^o$ is benzyl.

4. A process for making 4(5)-acetyl-2-methylimidazole which comprises

(a) reacting a compound of formula I

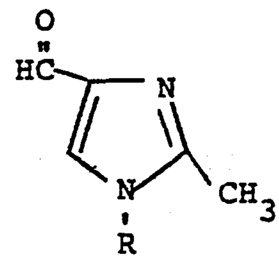

I

wherein R is benzyl or mono-substituted benzyl wherein the substitutent is chloro, bromo, fluoro, $(C_{1-4})$alkyl, $(C_{1-4})$alkoxy or phenyl,

with methylmagnesium chloride in a reaction-inert solvent to produce a compound of formula II

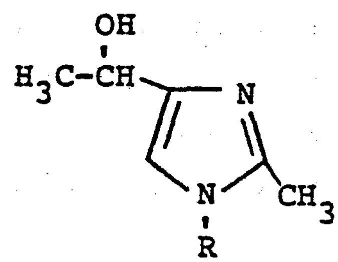

II

wherein R is as defined above;

(b) deblocking said formula II compound by catalytic hydrogenolysis to produce a compound of formula III

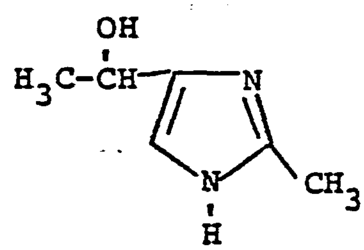

III;

(c) oxidizing said compound of formula III according to the process of claim 1.

5. A process for making a compound of formula IV

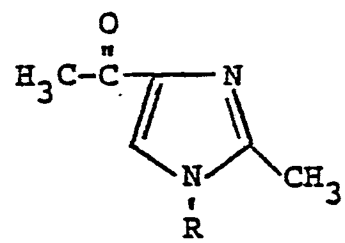

IV

wherein R is benzyl or mono-substituted benzyl wherein the substituent is chloro, bromo, fluoro, $(C_{1-4})$alkyl, $(C_{1-4})$alkoxy or phenyl;

which comprises oxidizing a compound of formula II

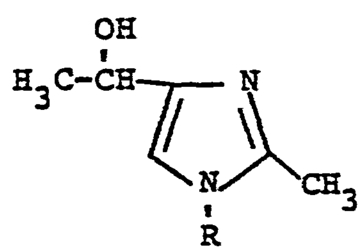

II

wherein R is as defined above, with manganese dioxide in a reaction-inert solvent.

6. A process for making 4(5)-acetyl-2-methylimidazole which comprises catalytically hydrogenolyzing a compound of formula IV wherein R is benzyl or mono-substituted benzyl by means of palladium-on-carbon and hydrogen.

European Patent Office

# EUROPEAN SEARCH REPORT

**0116205**

Application number

EP 83 30 7172

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-4 031 233 (KATHAWALA)<br>--- | 1-3,5 | C 07 D 233/60 |
| Y | US-A-3 812 189 (ENGLISH-BERKELHAMMER)<br>----- | 1-3,5 | |
| | | | TECHNICAL FIELDS SEARCHED (Int. Cl. 3) |
| | | | C 07 D 233/00 |

The present search report has been drawn up for all claims

| Place of search | Date of completion of the search | Examiner |
|---|---|---|
| THE HAGUE | 24-04-1984 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82